## Europäisches Patentamt
## European Patent Office
## Office européen des brevets

(11) Publication number: **0 074 193**
**B1**

(12) **EUROPEAN PATENT SPECIFICATION**

(45) Date of publication of patent specification: **29.10.86**

(21) Application number: **82304310.4**

(22) Date of filing: **16.08.82**

(51) Int. Cl.[4]: **C 07 C 29/14,** C 07 C 29/17, C 07 C 31/12, C 07 C 31/125, C 07 C 31/02

(54) Catalytic hydrogenation.

(30) Priority: **20.08.81 US 294519**

(43) Date of publication of application:
**16.03.83 Bulletin 83/11**

(45) Publication of the grant of the patent:
**29.10.86 Bulletin 86/44**

(84) Designated Contracting States:
**AT BE CH DE FR GB IT LI NL SE**

(56) References cited:
EP-A-0 008 767
BE-A- 892 958
DE-B-1 203 254
FR-A-1 600 072
FR-A-2 058 532
US-A-2 543 038

CHEMICAL ABSTRACTS, vol. 85, no. 7, Aug 16, 1976, p. 471, no. 45901r, Columbus, Ohio (US); A.V.UPADYSHEVA et al.: "Study of the composition of by-products formed during hydrogenation of 2-ethylhexen-2-al"

(73) Proprietor: **DAVY McKEE (LONDON) LIMITED
250, Euston Road
London NW1 2PG (GB)**

(72) Inventor: **Bradley, Michael William
52, Trefoil Wood Marton
Middlesbrough (GB)**
Inventor: **Harris, Norman
22, Grantham Road
Norton Cleveland (GB)**
Inventor: **Turner, Keith
12, The Avenue Fairfield
Stockton-on-Tees (GB)**

(74) Representative: **Eyles, Christopher Thomas et al
BATCHELLOR, KIRK & EYLES 2 Pear Tree Court
Farringdon Road
London, EC1R 0DS (GB)**

(56) References cited:

HYDROCARBON PROCESSING, vol. 59, no. 11, Nov 1980,pp. 93-102, Houston, Texas (US); B.CORNILS et al.: "2-EH: What you should know. Processes, feedstocks, production economics and uses for 2-ethylhexanol (2-EH( are reviewed"

## Description

This invention relates to catalytic hydrogenation more particularly to catalytic hydrogenation of aldehydes. Catalytic hydrogenation of aldehydes to form the corresponding alcohol is a well known reaction and is widely practised on a commercial scale. For example, n-butyraldehyde which is conventionally produced by oxo synthesis from propylene is catalytically hydrogenated on a large scale to form n-butanol, whilst 2-ethylpropylacrolein formed, for example by aldolisation of n-butyraldehyde to form 2-ethyl-3-hydroxyhexanal followed by dehydration, is reduced in considerable tonnage annually to form the plasticiser alcohol 2-ethylhexanol.

In such catalytic hydrogenation processes a by-product may be an ester. For example, when using certain hydrogenation catalysts, n-butyl butyrate can be detected in minor amounts in the reaction mixture resulting from catalytic hydrogenation of n-butyraldehyde. The formation of even a small percentage of esters, such as n-butyl butyrate, represents a serious loss in the potential yield of product alcohol, e.g. n-butanol. Such losses may render use of a particular hydrogenation catalyst, which otherwise has many advantageous properties, commercially unattractive.

Chem. Abs. 85:45901r (1976) discloses in outline the results of a study of the composition of by-products formed during hydrogenation of 2-ethylhexen-2-al(2-ethylpropylacrolein) over a nickel-chromium catalyst. Amongst these by-products is an unspecified amount of 2-ethylhexyl 2-ethylhexanoate.

Usually the amount of by-product ester formed increases with increasing temperature in the catalytic hydrogenation zone. Hence, in order to minimize formation of by-product ester, it is necessary to operate the catalytic hydrogenation zone at a relatively low temperature, which may in turn result in a somewhat reduced rate of catalytic hydrogenation and hence an increase in catalyst volume and in reactor size.

Moreover, since catalyst activity tends to decline in use as time passes, it is usually necessary to raise the reaction temperature gradually with passage of time in order to compensate for loss of catalytic hydrogenation activity. Such increase in temperature means that the amount of by-product ester formed increases so that eventually it becomes necessary to change the catalyst charge, because the level of by-product ester formation can no longer be tolerated, even though the original catalyst charge is still effective for aldehyde hydrogenation.

There is therefore a need to provide a process for catalytic hydrogenation of aldehydes to form the corresponding alcohols using hydrogenation catalysts, which otherwise tend to give rise to formation of by-product esters, wherein the effect of by-product ester formation is substantially eliminated or minimised, so as to maximise conversion of aldehyde to alcohol and to maximise the yield of alcohol based upon starting aldehyde.

There is also a need to extend the life of aldehyde hydrogenation catalysts whilst minimising losses of product through by-product ester formation.

Yet another need is to enable aldehyde hydrogenation catalysts to be used at higher than normal reaction temperatures without increasing losses through by-product ester formation.

The present invention accordingly seeks to provide a process for catalytic hydrogenation of aldehydes wherein potential loss of product alcohol by way of by-product ester formation is minimised. The invention further seeks to provide a process for the production of an alcohol by catalytic hydrogenation of an aldehyde in which at least a major part of the by-product ester formed is converted to product alcohol.

The present invention also seeks to provide a catalytic hydrogenation process in which an aldehyde is reduced to an alcohol with concomitant production of by-product ester wherein by-product ester is recovered and converted to product alcohol, thereby maximising the yield of product alcohol.

It also seeks to provide an aldehyde hydrogenation process in which catalyst life is extended and in which operation at higher than usual temperatures is permitted whilst minimizing losses of potential product through by-product ester formation.

According to the present invention there is provided a process for the production of an alcohol by catalytic hydrogenation of an aldehyde in a catalytic hydrogenation zone containing a charge of a hydrogenation catalyst effective for the hydrogenation of aldehydes in which the reaction mixture from the hydrogenation zone contains a minor amount of an ester containing twice as many carbon atoms as the aldehyde, characterised in that by-product ester is separated from the reaction mixture and a vaporous mixture containing the ester and hydrogen is contacted with a catalyst comprising a reduced mixture of copper oxide and zinc oxide at a pressure in the range of from about 75°C to about 300°C and at a pressure in the range of from about 0.1 kg/cm$^2$ (0.098 bar) absolute up to about 100 kg/cm$^2$ (98 bar) absolute.

Amongst hydrogenation catalysts that have been suggested for catalytic hydrogenation of aldehydes to give the corresponding alcohols, there may be mentioned catalysts such as nickel, cobalt, molybdenum sulphide, copper chromite, and the like. Such catalysts generally give rise to minor amounts of "heavies", i.e. materials having boiling points higher than the aldehyde starting material or the product alcohol. Amongst such "heavies" there may in some cases be present an ester; for example when hydrogenating n-butyraldehyde to n-butanol, the "heavies" by-products may include the ester n-butyl butyrate.

As a particular example of a hydrogenation catalyst that may be used in the catalytic hydrogenation zone and which is effective for the hydrogenation of aldehydes in the vapour phase, there may be mentioned a reduced mixture of copper oxide and zinc oxide. When such a catalyst is used for catalytic hydrogenation of an aldehyde, an ester is often detectable in the reaction product mixture. For example a minor amount of n-butyl butyrate may be present in the reaction product mixture obtained upon catalytic hydrogenation of n-butyraldehyde to n-butanol. Investigation has shown that, with this catalyst, the ester formation reaction is reversible and that the reaction product mixture may contain up to about 2 percent or more by weight of ester by-product. The mechanism of formation of the ester is not entirely clear but the currently available evidence is consistent with a mechanism whereby two molecules of n-butyraldehyde react together to form n-butyl butyrate by a Tischenko reaction.

The catalytic hydrogenation of aldehydes in the vapour phase using a reduced mixture of copper oxide and zinc oxide is described in more detail in European Patent Publication No. 0008767 (European Patent Application No. 79103181.8 filed 28th August 1979 by Union Carbide Corporation), the disclosure of which is herein incorporated by reference. The catalytic hydrogenation of the aldehyde may be conducted in a cooled tubular or multi-tubular reactor or in an adiabatic reactor. Usually it will be expedient to conduct the catalytic hydrogenation of the aldehyde to the alcohol in the presence of excess hydrogen. Typically the hydrogen:aldehyde molar ratio lies in the range of from about 5:1 to about 100:1, more preferably in the range of from about 20:1 to about 60:1.

The process of the invention is applicable to the hydrogenation of aldehydes or mixtures of aldehydes, particularly the vaporisable aldehydes. Such aldehydes preferably contain from 1 to about 20 carbon atoms and 1 or more aldehyde groups. Typical aldehydes include formaldehyde, acetaldehyde, propionaldehyde, n- and iso-butyraldehydes, n- and iso-valeraldehyde, n-hexaldehyde, n-heptaldehyde, n-octanal, 2-ethylhexanal, 2-ethylhex-2-enal (2-ethyl propyl acrolein), n-decanal, 2-ethylbutanal, propargyl aldehyde, acrolein, glyoxal, crotonaldehyde, furfural, aldol, hexahydrobenzaldehyde, alpha-citronellal, citral, chloral, trimethylacetaldehyde, diethylacetaldehyde, tetrahydrofurfural, phenylacetaldehyde, cinnamaldehyde, hydrocinnamaldehyde, and the like, as well as mixtures thereof.

The aldehyde or aldehydes used as starting material may be supplied to the catalytic hydrogenation zone as such or in admixture with inert vaporisable materials such as alcohols.

In the catalytic hydrogenation zone the hydrogen:aldehyde molar ratio preferably lies in the range of from about 2:1 to about 100:1, more preferably in the range of from about 5:1 to about 60:1. The temperature will usually lie in the range of from about 0°C to about 300°C, whilst the pressure is in the range of from about 0.1 kg/cm² (0.098 bar) absolute to about 100 kg/cm² (98 bar) absolute, is preferably not more than about 50 kg/cm² (49 bar) absolute, and is even more preferably in the range of from about 1 kg/cm² (0.98 bar) absolute to about 25 kg/cm² (24.5 bar) absolute.

When using a catalyst of the type described in the aforementioned European Patent Publication No. 0008767, it is essential that the aldehyde be supplied in vapour form to the catalytic hydrogenation zone. This requires that the reaction conditions in the catalytic hydrogenation zone shall be such that the aldehyde is always maintained above its dew point under the prevailing pressure and temperature conditions.

The reaction mixture exiting the catalytic hydrogenation zone in the process of the invention contains, in addition to product alcohol, a minor amount of ester by-product. If a mixture of aldehydes is used as starting material, then a mixture of ester by-products may be produced. For example, when using a mixture of n- and iso-butyraldehydes as starting material, a mixture of by-product esters is formed, viz. a mixture of n-butyl butyrate, iso-butyl butyrate, n-butyl iso-butyrate and iso-butyl iso-butyrate. Since the or each ester by-product has a molecular weight approximately twice that of the aldehyde and of the product alcohol, the boiling points of such esters are significantly higher than that of the aldehyde and of the product alcohol. Hence it will usually be convenient to recover the product alcohol or alcohols from the reaction mixture and to separate ester by-product or -products therefrom by distillation in each case. Distillation can be effected in one or more stages under normal, reduced or elevated pressure.

In the catalytic hydrogenation step one mole of hydrogen is consumed upon reduction or an aldehyde group —CHO to a primary alcohol group —CH₂OH. If the aldehyde starting material contains in addition ethylenic unsaturation, a further mole of hydrogen may be consumed by reduction of the ethylenic unsaturation in the catalytic hydrogenation zone. Hence two moles of hydrogen are consumed upon reduction of an unsaturated aldehyde such as 2-ethylhex-2-enal (2-ethylpropylacrolein) to the corresponding alcohol, e.g. 2-ethylhexanol. If the aldehyde contains acetylenic unsaturation, two moles of hydrogen are consumed upon reduction of the acetylenic linkage. For example catalytic hydrogenation of propargyl aldehyde to propanol will consume three moles of hydrogen.

According to a preferred procedure, by-product ester separated from the reaction product mixture exiting the catalytic hydrogenation zone is recycled to the inlet end of the catalytic hydrogenation zone. After a period of steady state operation the concentration of ester by-product in the reaction product mixture exiting the catalytic hydrogenation zone approaches an equilibrium concentration. Hence, if all the by-product ester is recycled to the inlet end of the catalytic hydrogenation zone, the ester concentration at the inlet end of the catalytic hydrogenation zone also approaches the equilibrium value so that the overall conversion of aldehyde to ester is minimised. If no bleed streams are taken to control the composition of the ester recycle stream, then the overall conversion of aldehyde to ester approaches zero.

As already mentioned, by-product ester is conveniently separated from the reaction product mixture exiting the catalytic hydrogenation zone by distillation. If the ester forms an azeotrope with one or more other components of the reaction product mixture, it is not necessary to separate this azeotrope which can be recycled as such to the inlet end of the catalytic hydrogenation zone.

In an alternative preferred procedure ester by-product separated from the reaction product mixture from the catalytic hydrogenation zone is contacted in a separate ester hydrogenolysis zone with the ester hydrogenolysis catalyst (i.e. the reduced mixture of copper oxide and zinc oxide).

Upon contact of the vaporous by-product ester with the catalyst comprising copper oxide and zinc oxide, whether this occurs in the catalytic hydrogenation zone or in a separate ester hydrogenolysis zone, hydrogenolysis occurs. Generally, both the acid moiety and alcohol moiety of the ester group appear as alcohol in the reaction product mixture. Thus, for example, n-butyl butyrate formed as by-product in the reduction of n-butyraldehyde is smoothly converted upon contact with the hydrogenolysis catalyst in the presence of hydrogen to two moles of n-butanol.

In the hydrogenolysis step the vaporous mixture to be contacted with the catalyst contains, in addition to by-product ester, hydrogen either alone or in admixture with other gases (desirably gases inert to the ester and the catalyst). Hence the gaseous mixtures containing hydrogen may include essentially inert gases such as nitrogen, or carbon monoxide.

DE—B—1203254 describes a process for the production of trans-dimethylolcyclohexan-(1,4) which involves hydrogenation of a mixture of esters of cis- and trans-hexahydrophthalic acid in the presence of a copper-containing catalyst at temperatures above 200°C, preferably in the range 240°C to 300°C and at elevated temperatures, typically 200 to 300 bar. In the Examples a solution of the esters in methanol is used in each case; under the reaction conditions used the hydrogenation process will operate as a liquid phase process.

In FR—A—1600072 high molecular weight polyalcohols are produced by hydrogenation of dimeric and/or oligomeric fatty acids or their esters over catalysts based on copper and/or zinc at temperatures of 150—300°C and pressures of 200—300 bar. Examples 2 and 3 each teach hydrogenation of a mixture of 3 parts of methanol and 1 part of a dimethyl ester of an unsaturated dimeric fatty acid containing 36 carbon atoms at a temperature of 250°C and 235°C respectively and at a pressure of 250 bar. In each case the ester solution will be hydrogenated in the liquid phase.

The ester hydrogenolysis conditions used in the process of the present invention are characterised by use of vapour phase conditions at pressures not exceeding about 100 kg/cm$^2$ absolute (98 bar).

A review article "2—EH: What you should know?" appeared on pages 93 to 102 of the November 1980 issue of "Hydrocarbon Processing". This discloses that, during aldolization of n-butyraldehyde, n-butyl butyrate can be formed as a by-product by simple and crossed Cannizzaro or Tischtschenko reactions (see equation (19) on page 98). The text goes on to state that, in this aldolization reaction, "A whole series of . . . C$_8$/C$_8$ esters can result . . .". Included within the term "C$_8$/C$_8$ esters" would be, for example, 2-ethylhexyl 2-ethylhexanoate. In the paragraph ". . By-product formation during hydrogenation" (beginning near the bottom of page 98, left-hand column) there is considerable discussion of possible by-products from this step, but the formation of esters at this stage is not recognised. In particular, there is no recognition that 2-ethylhexyl 2-ethylhexanoate would be a by-product of hydrogenation of the aldehyde concerned, i.e. 2-ethylhexenal, which yields the desired 2-ethylhexanol (2—EH). Page 200 describes the purification of the 2—EH product; in the second complete paragraph (page 100, right-hand column) it is stated in relation to Figure 6 that: "The bottom product of the 2—EH last runnings column 5 (Case A) or 6 (Case B) can be thermally treated before being recycled to the hydrogenation: otherwise C$_4^-$ or C$_8$ value products would be lost as heavy ends." Included in such heavy ends would be any C$_8$/C$_8$ ester (e.g. 2-ethylhexyl 2-ethylhexanoate) formed in the aldolization step. Thus the heavy ends are thermally treated to release "C$_4^-$ or C$_8$ value products"; in other words the thermal treatment is apparently strong enough to cause pyrolysis, effectively destroying any 2-ethylhexyl hexanoate present.

The hydrogenolysis step of the process of the invention is conducted at a temperature of between about 75°C and about 300°C; although in many cases the preferred temperature may be in the range of from about 150°C to about 200°C, in most cases it typically is between about 180°C and about 240°C. The total pressure is between about 0.1 kg/cm$^2$ (0.098 bar) absolute and about 100 kg/cm$^2$ (98 bar) absolute, preferably not more than about 50 kg/cm$^2$ (49 bar) absolute, and even more preferably between about 5 kg/cm$^2$ (4.9 bar) absolute and about 25 kg/cm$^2$ (24.5 bar) absolute.

The mixture of CuO and ZnO, before reduction, preferably contains from about 5 to about 95 percent by weight, typically from about 10 to about 70 percent by weight, of CuO and from about 95 to about 5 percent by weight, typically from about 90 to about 30 percent by weight, of ZnO. Hence the mixture may contain, for example, from about 20 to about 40 percent by weight of CuO and from about 60 to about 80 percent by weight of ZnO. A preferred mixture, for example, comprises from about 30 to about 36 percent by weight of CuO and from about 62 to about 68 percent by weight of ZnO. Other particularly preferred mixtures comprise from about 65 to about 85 percent by weight of CuO and from about 35 to about 15 percent by weight of ZnO, for example mixtures comprising from about 68 to about 75 percent by weight of CuO and from about 32 to about 25 percent by weight of ZnO. The hydrogenolysis catalyst may contain minor amounts of other materials such as carbon, sodium, titanium, zirconium, manganese, silica, diatomaceous earth, kieselguhr, and aluminium oxide. Such other materials do not usually comprise more than about 20 percent by weight calculated (except in the case of carbon) as oxide. In the case of sodium it is best not to exceed about 0.5 percent by weight, calculated as oxide. Hence other preferred catalysts include mixtures comprising from about 40 to about 50 weight percent each of CuO and ZnO and from 0 to about 20 weight percent of alumina. The catalyst is, however, preferably essentially free from other metals, particularly from metals of Group VIII of the Periodic Table, such as Fe, Co, Ni, Ru Rh, Pd, Os, Ir, and Pt, as well as from Group VIB metals, such as Cr, Mo, and W, from the metals Tc, Ag, Re, Au and Cd, and also from elements of atomic number 80 and above, e.g. Hg and Pb. By the term "essentially free" we mean that the catalyst contains not more than about 0.1 wt% (i.e. not more than about 1000 ppm), and preferably not more than about 250 ppm, of the element in question. The catalyst may be prepared by any of the methods known in the art of forming a composite of copper oxide and zinc oxide. The catalyst may be prepared by fixing the separate oxides, by coprecipitation of the oxalates, nitrates, carbonates, or acetates, followed by calcination. The coprecipitation method is preferred. Generally, the mixture of CuO and ZnO is reduced by hydrogen or carbon monoxide at a temperature in the range of between about 160°C and about 250°C for several hours, preferably for 8 to 24 hours, prior to contact with the vaporous mixture containing by-product ester and hydrogen. If the catalyst is charged in a pre-reduced form the period required for reduction can be reduced accordingly.

The mixture of CuO and ZnO is reduced prior to its use as catalyst in the hydrogenolysis step. Hydrogen or CO, as mixtures thereof, are generally mixed with a diluent gas such ·as steam, nitrogen, or combustion gas, to maintain the catalyst bed temperature and to carry away the heat of reduction.

Reduction of the mixture of CuO and ZnO is complete when no more hydrogen is being reacted as shown by analysis of the inlet and outlet hydrogen. Complete reduction of the mixture occurs when the total amount of water produced in the reduction is equal to the stoichiometric value of water which should be produced when a given amount of copper oxide is reduced to copper. This value is about 0.079 kg of water per kg of catalyst for a mixture containing 35 weight percent of CuO.

An inert carrier material may be included in the hydrogenolysis catalyst composition. The catalyst is generally formed into pellets, tablets, or any other suitable shape prior to use, by conventional techniques.

It is advantageous that the mixture of CuO and ZnO have an internal surface area of from about 25 to about 50 sq.m per gram. The internal surface area may be determined by the well-known BET method.

The reaction product mixture from the hydrogenolysis step may be separated from any excess hydrogen by condensation and the excess hydrogen can be compressed and recycled. This reaction product mixture comprises product alcohol in addition to a possible minor amount of unconverted ester by-product. This mixture is separated in any suitable manner, e.g. by distillation. The product alcohol may be used as recovered or it can be further purified in a conventional manner such as by fractional distillation. Preferably any unconverted by-product ester recovered is recycled to the hydrogenolysis stage.

In the hydrogenolysis step the partial pressure of the by-product ester may vary within wide limits, e.g. from about 0.05 kg/cm$^2$ (0.049 bar) or less up to about 10 kg/cm$^2$ (9.8 bar) or more. Care must however be taken to ensure that at all times the temperature of the vaporous mixture in contact with the hydrogenolysis catalyst is above the dew point of the ester under the prevailing pressure conditions.

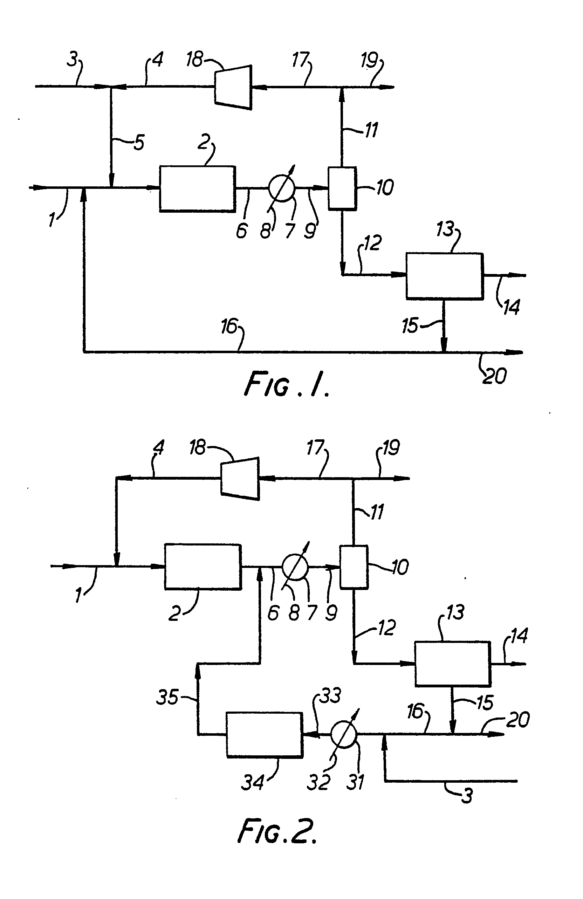

In order that the invention may be clearly understood and readily carried into effect, two preferred forms of plant utilising the process of the invention will now be described, by way of example only, with reference to the accompanying diagrammatic drawings, Figures 1 and 2 of which each illustrate a flow sheet of a plant for production of an alcohol by catalytic hydrogenation of an aldehyde.

It will be appreciated by those skilled in the art that, since the drawings are diagrammatic, various items of equipment which would be included in practice in an operational plant, such as valves, temperature measurement devices, pressure control devices, re-boilers and the like, have been omitted for the sake of clarity. Such standard items of equipment would be provided in accordance with conventional chemical engineering practice and form no part of the present invention.

Referring to Figure 1 of the drawings, a mixture of *n*- and *iso*- butyraldehydes in a molar ratio of about 6:1 is supplied through line 1 to a hydrogenation zone 2 containing a charge of a hydrogenation catalyst comprising a reduced mixture of CuO and ZnO. Make up hydrogen is supplied by way of line 3 and is mixed with recycled hydrogen in line 4, the mixed gases flowing on in line 5. The resulting vaporous mixture of hydrogen and aldehydes containing, for example, a hydrogen:aldehyde molar ratio of about 35:1 enters the inlet end of hydrogenation zone 2, which is maintained at a pressure of, for example, about 10 kg/cm$^2$ (9.8 bar) absolute, and at an inlet temperature of, for example, about 150°C.

Hydrogenation zone 2 may be an externally cooled tubular or multi-tubular reactor or an adiabatic or multi-stage adiabatic reactor with interstage cooling.

Hydrogenation of the aldehydes proceeds essentially to 100 percent conversion in hydrogenation zone 2. The vaporous reaction mixture exiting hydrogenation zone comprises, in addition to excess hydrogen, mainly *n*- and *iso*-butanols but also a minor proportion, usually in the range of from about 1 percent to about 2 percent by weight, of by-product esters (i.e. a mixture of *n*-butyl butyrate, together with lesser amounts of *iso*-butyl butyrate, *n*-butyl *iso*-butyrate and *iso*-butyl *iso*-butyrate), and traces of higher boiling compounds derived from selfcondensation products of *n*- and *iso*-butyraldehydes. This mixture passes by way of line 6 to a cooler 7 in which *n*- and *iso*-butanols and the other condensible components are condensed against cooling water supplied in line 8. The resulting gas/liquid mixture flows on in line 9 to a catchpot separator 10 from which the excess hydrogen is withdrawn in line 11, whilst the crude liquid product mixture is passed on in line 12 to separation zone 13 which conveniently takes the form of a distillation zone. Product alcohols, i.e. a mixture of *n*-butanol and *iso*-butanol are recovered from separation zone 13 as an overhead product in line 14, whilst by-product esters (e.g. *n*-butyl butyrate and isomers thereof) and "heavies" are recovered in the bottoms product in line 15 and are recycled to the inlet end of hydrogenation zone 2 in line 16. Any unconverted aldehyde is recovered together with the product alcohols in line 14 and can be separated therefrom in a downstream distillation zone (not shown) and recycled to the process.

The excess hydrogen in line 11 is recycled to the inlet end of hydrogenation zone 2 by way of line 17 by means of recycle gas compressor 18 and by way of line 4. A gas purge stream is taken through line 19 in order to control the level of impurities and inerts in the recirculating gas, whilst the build up of "heavies" in the liquid recycle line 16 is controlled by taking a liquid purge through line 20.

In Figure 2 the same reference numerals are used as are used in Figure 1 to denote like items of equipment. In this plant *n*-butyraldehyde is supplied in line 1 and the product alcohol is *n*-butanol, whilst the ester by-product is *n*-butyl butyrate. Make up hydrogen from line 3 is admixed with recycled *n*-butyl butyrate in line 16 and the resulting mixture is passed to vaporiser 31 which is heated by means of steam supplied in line 32. The resulting hydrogen/vaporous *n*-butyl butyrate mixture passes on by way of line 33 to a secondary hydrogenation zone 34 containing the same catalyst as that used in zone 2 of each of Figures 1 and 2. The inlet temperature to the hydrogenation zone is about 150°C and the pressure about 10 kg/cm² (9.8 bar) absolute. This secondary hydrogenation zone 34 may comprise an externally cooled tubular or multi-tubular reactor or a single bed adiabatic reactor or a multi-bed adiabatic reactor, possibly with intercooling between beds. Hydrogenolysis of *n*-butyl butyrate to *n*-butanol occurs in zone 34 and the resulting vaporous hydrogen/*n*-butanol mixture is recovered in line 35 and admixed with the vaporous mixture in line 6. The unreacted hydrogen from zone 34, which comprises by far the major portion of the make-up hydrogen, as well as the excess hydrogen supplied to the inlet end of zone 2, is passed to the inlet end of the *n*-butyraldehyde hydrogenation zone 2 by way of line 9, catchpot separator 10, lines 11 and 17, recycle gas compressor 18 and line 4, being admixed upstream from the *n*-butyraldehyde hydrogenation zone 2 with *n*-butyraldehyde supplied by way of line 1. Product separation, product recovery, and provision for gas and liquid purge streams are as in the plant of Figure 1.

The invention is further illustrated in the following Example:

Example

*n*-butyl butyrate was pumped at a rate of 3.8 ml/hr to an electrically heated gas/liquid mixing device to which hydrogen was also supplied at a controlled rate and pressure. The resulting vaporous mixture was passed through a lagged, electrically heated line to a pre-heating coil prior to passage through a tubular reactor packed with 146 ml of a powdered catalyst. Both the tubular reactor and the pre-heating coil were immersed in a molten salt bath which was heated to 174°C. The vaporous mixture exiting the reactor was passed through a water cooled condenser and the resulting condensate was collected in a water-cooled knock out pot. The exit gas pressure was controlled to 10.55 kg/cm² (10.35 bar) absolute. The non-condensed gases were then passed through a let-down valve, the gas flow being monitored downstream from this valve in a wet gas meter. A gas flow rate of 46.6 litres/hr (measured at atmospheric pressure) was maintained throughout the experiment.

The liquid condensate was analysed by gas chromatography using a 2 metre stainless steel column (6 mm outside diameter) packed with polyethylene glycol (nominal molecular weight 20,000) on Chromosorb PAW, a helium gas flow rate of 30 ml/minute and a flame ionisation detector. The instrument was fitted with a chart recorder having a peak integrator and was calibrated using a mixture of *n*-butanol and *n*-butyl butyrate of known composition. The condensate was shown to contain a mixture of 99.62 wt% butanol and 0.28 wt% *n*-butyl butyrate, corresponding to a 99.7% conversion with essentially 100% selectivity.

The catalyst used in this Example was charged to the reactor as a co-precipitated mixture of CuO and ZnO containing 33 ± 3% CuO and 65 ± 3% ZnO having a particle size in the range of 1.2 mm to 2.4 mm and an internal surface area of about 45 sq. m. per gram. This was pre-reduced in the reactor using a 5 vol% $H_2$ gas mixture at 200°C for 17 hours followed by pure hydrogen at 200°C for 8 hours, the gas flow rate in each case being about 20 litres/hr (measured at atmospheric pressure using the wet gas meter) and the gas pressure being 10.55 kg/cm$^2$ (10.35 bar) absolute. After this pre-reduction stage the catalyst was at all times maintained in a hydrogen-containing atmosphere.

## Claims

1. A process for the production of an alcohol by catalytic hydrogenation of an aldehyde, which preferably contains from 1 to about 20 carbon atoms, in a catalytic aldehyde hydrogenation zone containing a charge of a hydrogenation catalyst effective for the hydrogenation of aldehydes, followed by recovery of a reaction mixture containing, in addition to product alcohol, a minor amount of a by-product ester containing twice as many carbon atoms as the aldehyde, characterised in that by-product ester is separated from the reaction mixture, and a vaporous mixture comprising by-product ester and hydrogen is contacted with a catalyst comprising a reduced mixture of copper oxide and zinc oxide at a temperature in the range of from about 75°C to about 300°C and at a pressure in the range of from about 0.1 kg/cm$^2$ (0.098 bar) absolute up to about 100 kg/cm$^2$ (98 bar) absolute.

2. A process according to claim 1, characterised in that the aldehyde is selected from *n*-butyraldehyde, *iso*-butyraldehyde, and mixtures thereof and the by-product ester is selected from *n*-butyl butyrate, *iso*-butyl butyrate, *n*-butyl *iso*-butyrate and *iso*-butyl *iso*-butyrate, and mixtures thereof.

3. A process according to claim 1, characterised in that the aldehyde is propionaldehyde and the by-product ester is *n*-propyl propionate.

4. A process according to claim 1, characterised in that the aldehyde is 2-ethylpropylacrolein and the by-product ester is 2-ethylhexyl 2-ethylhexanoate.

5. A process according to any one of claims 1 to 4, characterised in that the hydrogenation catalyst of the catalytic aldehyde hydrogenation zone comprises a reduced mixture of copper oxide and zinc oxide.

6. A process according to claim 5, characterised in that the catalytic aldehyde hydrogenation zone is maintained at a temperature in the range of from about 75°C to about 300°C and at a pressure in the range of from about 0.1 kg/cm$^2$ (0.098 bar) absolute up to about 100 kg/cm$^2$ (98 bar) absolute.

7. A process according to claim 5 or claim 6, characterised in that by-product ester separated from the reaction mixture is recycled to the upstream end of the catalytic aldehyde hydrogenation zone.

8. A process according to any one of claims 1 to 6, characterised in that by-product ester separated from the reaction mixture is contacted in the vapour phase in admixture with hydrogen in a secondary catalytic hydrogenation zone with a catalyst selected from (a) those consisting essentially of a reduced mixture of copper oxide and zinc oxide and (b) those consisting essentially of a reduced mixture of copper oxide and zinc oxide and a minor amount, not exceeding about 20 percent by weight, of at least one material selected from carbon, silica, diatomaceous earth, kieselguhr and aluminium oxide, at a partial pressure of the by-product ester of at least about 0.05 kg/cm$^2$ (0.049 bar).

9. A process according to claim 8, characterised in that make-up hydrogen is admixed with by-product ester separated from the reaction mixture, the excess hydrogen issuing from the secondary catalytic hydrogenation zone being supplied as make up hydrogen to the catalytic aldehyde hydrogenation zone.

10. A process according to any one of claims 1 to 9, characterised in that the hydrogen:aldehyde ratio in the catalytic aldehyde hydrogenation zone lies in the range of from about 2:1 to about 100:1.

## Patentansprüche

1. Verfahren zur Herstellung eines Alkohols durch katalytische Hydrierung eines Aldehyds, der vorzugsweise 1 bis etwa 20 Kohlenstoffatome enthält, in einer katalytischen, eine Füllung eines für die Aldehydhydrierung wirksamen Hydrierkatalysators enthaltenden Aldehyd-Hydrierzone mit nachfolgender Gewinnung eines Reaktionsgemisches, das außer dem Produktalkohol eine geringere Menge eines doppelt so viele Kohlenstoffatome wie der Aldehyd enthaltenden Nebenprodukt-Esters enthält, dadurch gekennzeichnet, daß man den Nebenprodukt-Ester von dem Reaktionsgemisch abtrennt und ein dampfförmiges, den Nebenprodukt-Ester und Wasserstoff enthaltendes Gemisch bei einer Temperatur in dem Bereich von etwa 75°C bis etwa 300°C und bei einem Druck in dem Bereich von etwa 0,1 kg/cm$^2$ (0,098 bar) absolut bis etwa 100 kg/cm$^2$ (98 bar) absolut mit einem ein reduziertes Gemisch von Kupferoxid und Zinkoxid aufweisenden Katalysator in Berührung bringt.

2. Verfahren nach Anspruch 1, dadurch gekennzeichnet, daß man den Aldehyd unter n-Butyraldehyd, iso-Butyraldehyd und deren Mischungen auswählt und den Nebenprodukt-Ester unter n-Butylbutyrat, iso-Butylbutyrat, n-Butyl-isobutyrat und iso-Butylisobutyrat und deren Mischungen auswählt.

3. Verfahren nach Anspruch 1, dadurch gekennzeichnet, daß der Aldehyd Propionaldehyd und der Nebenprodukt-Ester n-Propylpropionat ist.

4. Verfahren nach Anspruch 1, dadurch gekennzeichnet, daß der Aldehyd 2-Äthylpropylacrolein und der Nebenprodukt-Ester 2-Äthylhexansäure-2-äthylhexylester ist.

5. Verfahren nach einem der Ansprüche 1 bis 4, dadurch gekennzeichnet, daß der Hydrierkatalysator der katalytischen Aldehyd-Hydrierzone ein reduziertes Gemisch von Kupferoxid und Zinkoxid aufweist.

6. Verfahren nach Anspruch 5, dadurch gekennzeichnet, daß man die katalytische Aldehyd-Hydrierzone auf einer Temperatur in dem Bereich von etwa 75°C bis etwa 300°C und bei einem Druck in dem Bereich von etwa 0,1 kg/cm$^2$ (0,098 bar) absolut bis etwa 100 kg/cm$^2$ (98 bar) absolut hält.

7. Verfahren nach Anspruch 5 oder 6, dadurch gekennzeichnet, daß man den von dem Reaktionsgemisch abgetrennten Nebenprodukt-Ester zu der Anströmseite der katalytischen Aldehyd-Hydrierzone zurückführt.

8. Verfahren nach einem der Ansprüche 1 bis 6, dadurch gekennzeichnet, daß man den von dem Reaktionsgemisch abgetrennten Nebenprodukt-Ester in der Dampfphase im Gemisch mit Wasserstoff in einer sekundären katalytischen Hydrierzone bei einem Partialdruck des Nebenprodukt-Esters von wenigstens etwa 0,05 kg/cm$^2$ (0,049 bar) mit einem Katalysator in Berührung bringt, den man unter (a) jenen, die im wesentlichen aus einem reduzierten Gemisch von Kupferoxid und Zinkoxid bestehen, und (b) jenen, die im wesentlichen aus einem reduzierten Gemisch von Kupferoxid und Zinkoxid und einer geringeren, etwa 20 Gew.-% nicht übersteigenden Menge wenigstens eines unter Kohlenstoff, Siliziumdioxid, Diatomeenerde, Kieselgur und Aluminiumoxid ausgewählten Materials bestehen, auswählt.

9. Verfahren nach Anspruch 8, dadurch gekennzeichnet, daß man Ergänzungswasserstoff mit dem von dem Reaktionsgemisch abgetrennten Nebenprodukt-Ester mischt, wobei der aus der sekundären katalytischen Hydrierzone austretende Wasserstoffüberschuß als Ergänzungswasserstoff der katalytischen Aldehyd-Hydrierzone zugeführt wird.

10. Verfahren nach einem der Ansprüche 1 bis 9, dadurch gekennzeichnet, daß das Wasserstoff/Aldehyd-Verhältnis in der katalytischen Aldehyd-Hydrierzone in dem Bereich von etwa 2:1 bis etwa 100:1 liegt.

**Revendications**

1. Procédé pour la production d'un alcool par hydrogénation catalytique d'un aldéhyde qui, de préférence, contient de 1 à environ 20 atomes de carbone, dans une zone d'hydrogénation catalytique d'aldéhyde contenant une charge d'un catalyseur d'hydrogénation efficace pour l'hydrogénation des aldéhydes, puis la récupération d'un mélange réactionnel contenant, en plus de l'alcool produit, une petite quantité d'un ester formé comme sous-produit contenant deux fois plus d'atomes de carbone que l'aldéhyde, caractérisé en ce que l'ester formé comme sous-produit est séparé d'avec le mélange réactionnel, et un mélange à l'état de vapeur, comprenant l'ester formé comme sous-produit et de l'hydrogène, est mis en contact avec un catalyseur comprenant un mélange réduit d'oxyde de cuivre et d'oxyde de zinc à une température dans la gamme d'environ 75°C à environ 300°C et à une pression dans la gamme d'environ 0,1 kg/cm$^2$ (0,098 bar) absolu à environ 100 kg/cm$^2$ (98 bars) absolus.

2. Procédé selon la revendication 1, caractérisé en ce que l'aldéhyde est choisi parmi le n-butyraldéhyde, l'isobutyraldéhyde et leurs mélanges, et l'ester formé comme sous-produit est choisi parmi le butyrate de n-butyle, le butyrate d'isobutyle, l'isobutyrate de n-butyle et l'isobutyrate d'isobutyle et leurs mélanges.

3. Procédé selon la revendication 1, caractérisé en ce que l'aldéhyde est le propionaldéhyde et l'ester formé comme sous-produit est le propionate de n-propyle.

4. Procédé selon la revendication 1, caractérisé en ce que l'aldéhyde est la 2-éthylpropylacroléine et l'ester formé comme sous-produit est le 2-éthylhexanoate de 2-éthylhexyle.

5. Procédé selon l'une quelconque des revendications 1 à 4, caractérisé en ce que le catalyseur d'hydrogénation de la zone d'hydrogénation catalytique de l'aldéhyde comprend un mélange réduit d'oxyde de cuivre et d'oxyde de zinc.

6. Procédé selon la revendication 5, caractérisé en ce que la zone d'hydrogénation catalytique de l'aldéhyde est maintenue à une température dans la gamme d'environ 75°C à environ 300°C et à une pression dans la gamme d'environ 0,1 kg/cm$^2$ (0,098 bar) absolu à environ 100 kg/cm$^2$ (98 bars) absolus.

7. Procédé selon la revendication 5 ou la revendication 6, caractérisé en ce que l'ester formé comme sous-produit séparé d'avec le mélange réactionnel est recyclé à l'extrémité d'amont de la zone d'hydrogénation catalytique de l'aldéhyde.

8. Procédé selon l'une quelconque des revendications 1 à 6, caractérisé en ce que l'ester formé comme sous-produit séparé d'avec le mélange réactionnel est mis en contact en phase vapeur en mélange avec de l'hydrogène dans une zone d'hydrogénation catalytique secondaire avec un catalyseur choisi parmi (a) ceux consistant essentiellement en un mélange réduit d'oxyde de cuivre et d'oxyde de zinc et (b) ceux consistant essentiellement en un mélange réduit d'oxyde de cuivre et d'oxyde de zinc et d'une petite quantité, ne dépassant pas environ 20% en poids, d'au moins une matière choisie parmi le carbone, la silice, la terre de diatomées, le kieselguhr et l'oxyde d'aluminium, sous une pression partielle de l'ester formé comme sous-produit d'au moins environ 0,05 kg/cm$^2$ (0,049 bar).

9. Procédé selon la revendication 8, caractérisé en ce que l'hydrogène d'appoint est mélangé avec l'ester formé comme sous-produit séparé d'avec le mélange réactionnel, l'excès d'hydrogène sortant de la zone d'hydrogénation catalytique secondaire étant fourni comme hydrogène d'appoint à la zone d'hydrogénation catalytique de l'aldéhyde.

10. Procédé selon l'une quelconque des revendications 1 à 9, caractérisé en ce que le rapport hydrogène/aldéhyde dans la zone d'hydrogénation catalytique de l'aldéhyde se situe dans la gamme d'environ 2/1 à environ 100/1.

Fig . I.

Fig. 2.